# EUROPEAN PATENT SPECIFICATION

(11) **EP 2 429 490 B1**
(45) Date of publication and mention of the grant of the patent: **23.11.2016**
(21) Application number: 10722600.3
(22) Date of filing: 12.05.2010
(51) Int. Cl.: A61K 9/06, A61K 47/10, A61K 31/58

(54) **OIL-IN-WATER EMULSION OF MOMETASONE**
ÖL-IN-WASSER-EMULSION VON MOMETASON
ÉMULSION HUILE-DANS-EAU DE MOMÉTASONE

(30) Priority: 12.05.2009 DK 200900602
(43) Date of publication of application: 21.03.2012
(73) Proprietor: Galenica AB, 205 12 Malmö (SE)
(72) Inventor: HANSSON, Henri, S-255 91 Helsingborg (SE); MORÉN, Anna Karin, S-217 74 Malmö (SE)
(74) Representative: Chas. Hude A/S
(86) International application number: PCT/EP2010/002929
(87) International publication number: WO 2010/130435

(56) References cited:
- EP-A1- 1 886 686
- WO-A-91/08733
- WO-A-03/097070
- WO-A-2008/126076
- US-A- 5 696 105
- US-A- 6 075 056

## Description

### Field of the invention

The present invention provides a novel pharmaceutical composition of mometasone or a pharmaceutically acceptable derivate thereof in the form of an oil-in-water emulsion, notably a cream. The composition has excellent stability and therapeutic effect.

### Background of the invention

The exceptionally poor solubility of mometasone furoate has delayed the development of efficacious, economic and cosmetically elegant topical formulation. The existing mometasone creams on the market today are all based on water-in-oil emulsions.

One of the most challenging tasks for formulators is to incorporate poorly water-soluble drugs into effective products. Improving the solubility of the lipophilic drugs is considered to improve the bioavailability of the product. Therefore, formulations where the active substance is in a dissolved state are generally preferred. Normally the active substance is in a solubilized form when permeating the skin. Therefore it is also generally considered as an advantage when the active substance is in a solubilized form in the topical formulation in order to obtain a suitable therapeutic response.

### Background of the invention

US patent No. 4,808,610 (Schering Corp) and US patent No. 7,312,207 (Taro Pharmaceuticals) relate to mometasone containing compositions for topical use, wherein the composition is in the form of a water-in-oil (w/o) emulsion.

WO 91/08733 (Schering Corp) relates to an oil-in-water (o/w) emulsion comprising a lipophilic active drug substance (e.g. mometasone). The examples show the necessity of using N-methyl-2-pyrrolidone in order to enhance the vasoconstrictor effect. In the examples propylene glycol is used in a concentration of 10% w/w.

WO 2008/126076 (Perrigo Israel Pharmaceuticals Ltd.) relates to a low-dose mometasone formulation. Exemplary formulations are creams containing 0.075% mometasone, a polyol, a gelling agent, an oily phase and water. A low-dose mometasone formulation is desired in order to reduce the toxicity of a mometasone formulation. It is believed that a formulation of WO 2008/126076 has a relatively low systemic steroid absorption. No *in vivo* studies are reported.

US 6,075,056 relates to composition containing two different active drug substances, namely a steroid and an antifungal agent. The composition may be formulated as a cream and propylene glycol may be employed preferably in concentrations of 3-15%.

### Detailed description of the invention

The present invention provides an oil-in-water emulsion (o/w) containing mometasone as an active drug substance. Moreover, the emulsion contains a C₄ alkane-diol, wherein the two hydroxyl groups are linked to two different carbon atoms and wherein the C₄ alkane may be a straight chain or branched. The C₄ alkane-diol may be a butanediol (butylene glycol) such as 1,3-butane-diol. Preferred substance is butylene glycol.

As it appears from the examples herein, an o/w emulsion of the present invention provides a bioavailability and a therapeutic effect of mometasone that is comparable to the marketed w/o emulsion (Elocon® cream). As briefly discussed in the introduction, the general view has been that in order to achieve a suitable therapeutic response it is of utmost importance to have the active drug dissolved or solubilised in the composition. Considering the lipid nature of mometasone furoate it has thus, until now, been considered to have mometasone furoate dissolved in the formulation. However, as discussed below, the present inventors have found that it is not necessary to have all mometasone dissolved in an o/w emulsion in order to achieve a suitable therapeutic effect.

The present inventors have found that using mometasone (e.g. mometasone furoate) and a C₄ alkane-diol (notably butylene glycol) in a relatively high concentration (from 20% to 45% w/w) enables the formulation of an o/w emulsion, wherein mometasone is at least partly dissolved in the formulation (as appears from the examples herein, mometasone furoate is partly dissolved in the formulation). Moreover, it seems to be possible to obtain an o/w emulsion with a similar therapeutic effect to that seen with Elocon® cream, i.e. the o/w emulsion only need to be applied once daily. To this end and as indicated by the results reported herein, a suitable weight ratio between alkane diol and water in the o/w emulsion seems to be important for the once daily administration. It seems as if a too large or a too low ratio will not result in the desired effect and/or the desired bioavailability. Another factor that seems to be of importance is the presence of mometasone (e.g. mometasone furoate) in micronised from. Normally, mometasone is not dissolved (or only partly dissolved) in a composition of the invention. The results reported herein indicates the importance of having undissolved mometasone in micronised from. Thus, in general, 100% of the mometasone particles has a particle size of at the most 20 µm, 99% has a particle size of at the most 15 µm, and 80% has a particle size of at the most 5 µm when measured by means of a laser scattering method. When determining the particle size by means of light microscopy (which is the method preferred, when the particle size is measured in the final composition), no particle of mometasone should exceed 40 µm. Most particles (more than 80% and visually evaluated in the microscope) have a size between 10 and 20 µm. Eventual particle growth over time in the composition should not result in any particles exceeding 50 µm, when measured with light microscopy. Other factors may also influence the results such as the nature of the other ingredients employed. However, the two most important factors in compositions of the present invention seem to be the weight ratio and particle size as discussed above.

For propylene glycol, a suitable weight ratio between propylene glycol and water is from about 1:1 to about 1:3. It is contemplated that a similar suitable weight ratio between butylene glycol and water is from about 1:1 to about 1:3. As illustrated in the examples, it is possible to obtain an emulsion with balanced content of mometasone (e.g. mometasone furoate). a C₄ alkane-diol and water that is bioequivalent with the w/o Elocon® cream.

A composition of the invention contains two phases, an aqueous phase, which is the continuous phase and an oil phase, which is the dispersed phase that is homogeneously distributed in the continuous phase (i.e. as generally seen in o/w emulsions). Moreover, the active drug substance, mometasone (e.g. mometasone furoate), is partly dissolved and partly present in the form of fine particles, notably in micronized form.

More specifically, the present invention provides an oil-in-water emulsion comprising mometasone (M) or a pharmaceutically acceptable derivative thereof and a C₄ alkane-diol, which, if relevant, may be linear or branched, and the concentration of the C₄ alkane-diol is from 20% w/w to 45% w/w, from 15% w/w to 30% w/w or from 20% to 30% w/w. The use of butylene glycol in o/w emulsions of mometasone is illustrated in the Examples herein It is contemplated that other alkane-diols as well may be used in combination with butylene glycol. Propylene glycol and butylene glycol are normally regarded as relatively non-toxic substance with minimally irritant effect. Pentylene glycol and hexylene glycol have even lower irritative effects after topical administration and, accordingly, it is contemplated that such substances could be used in higher concentrations than propylene glycol and butylene glycol (i.e. from about 20% w/w to 50% w/w, notably from about 30% w/w to 45% w/w). If pentylene glycol or hexylene glycol is used in combination with butylene glycol the concentration of pentylene glycol or hexylene glycol is from about 1% w/w to about 20% w/w (notably from about 5% w/w to about 20% w/w).

As evident from the examples herein, incorporation of the C₄ alkane-diol is very important in order to obtain the desired effect, and not only is it the incorporation of any amount of the C₄ alkane-diol in the emulsion, but also the concentration of the C₄ alkane-diol, either expressed as the concentration in the total emulsion, or, more specific, expressed as a ratio between the C₄ alkane-diol and water. When an oil-in-water emulsion of the invention is applied to the skin, the water in the composition is subject to evaporation. Thus, not only the weight ratio between C₄ alkane-diol and water in the composition may be important, but also the ratio between the C₄ alkane-diol and mometasone or a pharmaceutically acceptable derivative thereof may have impact on the therapeutic result. One or more of these factors are contemplated to be decisive for whether an emulsion can be obtained with suitable properties with respect to therapeutic effect.

In the present context, the term "mometasone" includes mometasone or pharmaceutically acceptable derivatives thereof. Thus, the term includes mometasone as such as well as suitable ester derivatives such as esters with organic acid normally used in pharmaceutics including the furoate ester. Moreover, "mometasone or pharmaceutically acceptable derivatives thereof" includes any form such as anhydrous form, hydrate forms including the monohydrate, solvates other than hydrates etc., as well as amorphous, polymorph and crystalline forms thereof. In the present context, all calculation relating to "mometasone or pharmaceutically acceptable derivatives thereof' is based on mometasone furoate. Accordingly, if another derivative is employed, an equivalent amount of mometasone furoate must be calculated based on the molecular weights of the derivative and the mometasone furoate.

The concentration of mometasone (calculated as mometasone furoate) in an emulsion of the present invention may be from about 0.01 % to 2% w/w, normally from 0.05% to 0.2% w/w, from 0.075% to 0.2% w/w such as about 0.1 % w/w.

In an emulsion according to the invention, mometasone (mometasone furoate) is not fully dissolved. Accordingly, a part of mometasone or a pharmaceutically acceptable derivative thereof is present in undissolved form, typically from about 25 to about 35 % w/w of the total amount of mometasone present in the emulsion. In order to improve the dispersibility of the undissolved mometasone as well as the absorption rate, mometasone should be employed in micronised form. As it appears from the examples herein, mometasone, e.g. mometasone furoate, should be employed in micronised form, wherein 100% has a particle size of at the most 20 µm, 99% has a particle size of at the most 15 µm, and 80% has a particle size of at most 5 µm (when determined by laser microscopy). It is contemplated that during the manufacturing process, as described herein, mometasone furoate may partly dissolve, but is not fully dissolved. This seems to be of importance in order to control the particle size in the final composition. If mometasone is fully dissolved during the manufacturing method, a risk will occur that the mometasone that precipitates in the composition has a too large particle size. Accordingly, it is envisaged that the mean particle size (and/or particle size distribution) of mometasone furoate employed is important in order to obtain reproducible therapeutic results.

The C₄ alkane-diol is a butylene glycol. A specific example is butane-1,3-diol. It is envisaged that other diols in combination with butylene glycol also may be suitable for use in the present context provided that it is suitable and safe for topical use.

As mentioned above, the concentration of the C₄ alkane-diol (abbreviated "alkane-diol" herein) in an emulsion of the invention is from about 20% to about 45% w/w. As discussed above, the concentration depends on the specific C₄ alkane-diol used. In general, the concentration of the C₄ alkane-diol, notably butylene glycol, is from about 20% to about 40% w/w such as from about 20% to about 30% w/w.

As mentioned above, the weight ratio between the alkane-diol and water seems to be very important in order to achieve the desired therapeutic effect. Moreover, the weight ratio between butylene glycol and mometasone (M) may also be a useful parameter to decide the amount of alkane-diol in an emulsion of the invention. In the following table calculations of suitable ranges of the ratio are given. As seen from the table, the lower limit is normally not less than 7.5, such as in a range of from 7.5 to 100 and the upper limit is normally 4500 or less such as in a range of from 600 to 4500. Normally, the ratio is 75 or more such as from 75 to 900 or from about 150 to about 450, and M is calculated as mometasone furoate. Specifically, the weight ratio between the alkane-diol and mometasone (M) is from 200 to 300, such as 200, 225, 250, 275 or 300, and M is calculated as mometasone furoate.

| Alkane-diol % w/w I | Mometasone (calculated as mometasone furoate) % w/w II | Ratio I/II |
|---|---|---|
| 20-40 | 0.01-2 | 10-4000 |
| 20-30 | 0.01-2 | 10-3000 |
| 15-45 | 0.05-1 | 15-900 |
| 20-40 | 0.05-1 | 20-800 |
| 20-30 | 0.05-1 | 20-600 |
| 15-45 | 0.05-0.5 | 30-900 |
| 20-40 | 0.05-0.5 | 40-800 |
| 20-30 | 0.05-0.5 | 40-600 |
| 20-40 | 0.05-0.2 | 100-800 |
| 20-30 | 0.05-0.2 | 100-600 |

Generally, the weight ratio between butylene glycol and mometasone (M) is 75 or more such as, e.g. 100 or more or 150 or more, such as, e.g., from 75 to 900, from about 150 to about 500, and M is calculated as mometasone furoate.

Specifically, the weight ratio between butylene glycol and mometasone (M) is from 150 to 450, from 200 to 450, and M is calculated as mometasone furoate.

In particular, the weight ratio between butylene glycol and mometasone (M) is from 200 to 300, such as 200, 225, 250, 275 or 300, and M is calculated as mometasone furoate.

Moreover, the weight ratio between the alkane-diol, notably butylene glycol, and water is indicative of whether a suitable emulsion is obtained (with respect to therapeutic activity). Thus, the weight ratio (BG:W) is normally from 1:1 to about 1:3 such as from about 1:1.5 to about 1:2.5 or from 1:1.75 to 1:2.25. In the examples a suitable ratio is found to be about 1:2.0 or 1:2.1 when 25% alkane-diol is employed and from about 1:2.8 to 1:2.9 when 20% alkane-diol is employed. In those cases, where the alkane-diol is pentylene glycol or hexylene glycol, the concentration of such an alkane-diol in the composition is normally higher such as 30-45% w/w. In those cases, the ratio between the alkane-diol and water is from about 1:0.7 to about 1:1.5.

The above-mentioned paragraphs apply *mutatis mutandis* when a mixture of butylene glycol with one or more C₃-C₆ alkane-diols is employed (i.e. same concentration ranges, same weight ratio ranges etc.).

In the literature, combinations of steroids with other active ingredients are described. However, as is evident from the examples herein, the invention is directed to an oil-in-water emulsion, wherein mometasone or a pharmaceutically acceptable derivative thereof is the sole therapeutically active ingredient. Further active ingredients may be added provided that the bioavailability and therapeutic effect of mometasone are not negatively affected. Moreover, further active ingredients may not have any negative effect on the stability of the o/w emulsion.

As mentioned above, an emulsion is established by mixing an aqueous phase and an oil phase. In an oil-in-water emulsion, the oil is present in droplets homogeneously dispersed in the aqueous phase. In order to stabilize the emulsion against phase separation, surface active agents or emulsifying agents are added.

In an emulsion of the present invention, the oil phase comprises an oil selected from a group consisting of vegetable oils and fats, animal oils and fats, mineral oils, ester oils, silicon oils or waxes. Notably, the oil/fat is a vegetable oil/fat such as coconut oil, olive oil, sunflower oil and canola oil etc. Fats can be defined as bulk storage material produced by plants, animals and microorganisms that contain aliphatic moieties, such as fatty acid derivatives. These are mainly, but not entirely, mixtures of triglycerols (triglycerides) and are known as oils or fats depending on whether they are liquid or solid at room temperature. In the present context, the term "oil" also includes "fat" and oil/fat may also be produced synthetically or semi-synthetically.

The concentration of the oil/fat in the emulsion is from about 3 to about 30% w/w, notably from about 5 to about 15% w/w.

As mentioned above, stabilization of an oil-in-water emulsion according to the invention may suitably be carried out by adding one or more emulsifying agents. An emulsion of the present invention may therefore comprise one or more emulsifying agents. As seen from the examples herein use of three emulsifying agents having a HLB (hydrophilic-lipophilic balance) in the range 3-20, one with a high HLB, i.e. a HLB of, from about 11 - 20 and two with a low HLB, i.e. a HLB of, from about 3 - 11, gives the desired result with respect to stability without compromising the therapeutic effect.

Suitable emulsifying agents for use in a composition of the invention may be selected from the group consisting of glycerol alkyl esters, macrogol alkyl esters, polyoxyethyleneglycol alkyl esters, fatty acids, polyoxyethylene sorbitan esters, polyoxyethylene alkyl ethers, galactolipids.

Specific emulsifying agents for use in a composition of the invention are glycerol monostearate 40-55, macrogol stearate, and stearic acid.

The concentration of each emulsifier when present in an emulsion according to the present invention ranges from 1-5% w/w.

Moreover, an oil-in-water emulsion according to the invention may comprise a viscosity-increasing agent. Viscosity-increasing agents suitable for use in an emulsion may be selected from the group consisting of fatty alcohols (concentration range 5-15% of total emulsion).

As seen from the examples herein, a suitable viscosity-increasing agent is cetostearyl alcohol.

In general, the concentration of the viscosity-increasing agent in the form of a fatty alcohol ranges from 5% to 15 % w/w.

An emulsion of the invention is intended for topical use, i.e. as a cream to apply on the skin. Accordingly, pH may be adjusted to a skin-friendly value taking into consideration stability issues relating to mometasone. A suitable pH is below 6 such as from about 3 to about 6 or from about 4.0 to about 5.0. pH may be adjusted by use of one or more pH adjusting agent, which is selected from the group consisting of hydrochloric acid, phosphoric acid, sodium hydroxide, citrate buffer, phosphate buffer, phthalate buffers, acetate buffers, succinate buffers. In order to arrive at a pH of about 4.0-5.0, a citrate buffer has proved to be suitable.

Moreover, an emulsion of the present invention may contain one or more fragrances. Addition of a preservative agent is normally not required as the C₄ alkane-diol itself has antimicrobial effect when the C₄ alkane-diol is added in a sufficient concentration.

More specifically, the invention relates to an oil-in-water emulsion having one of the following compositions:

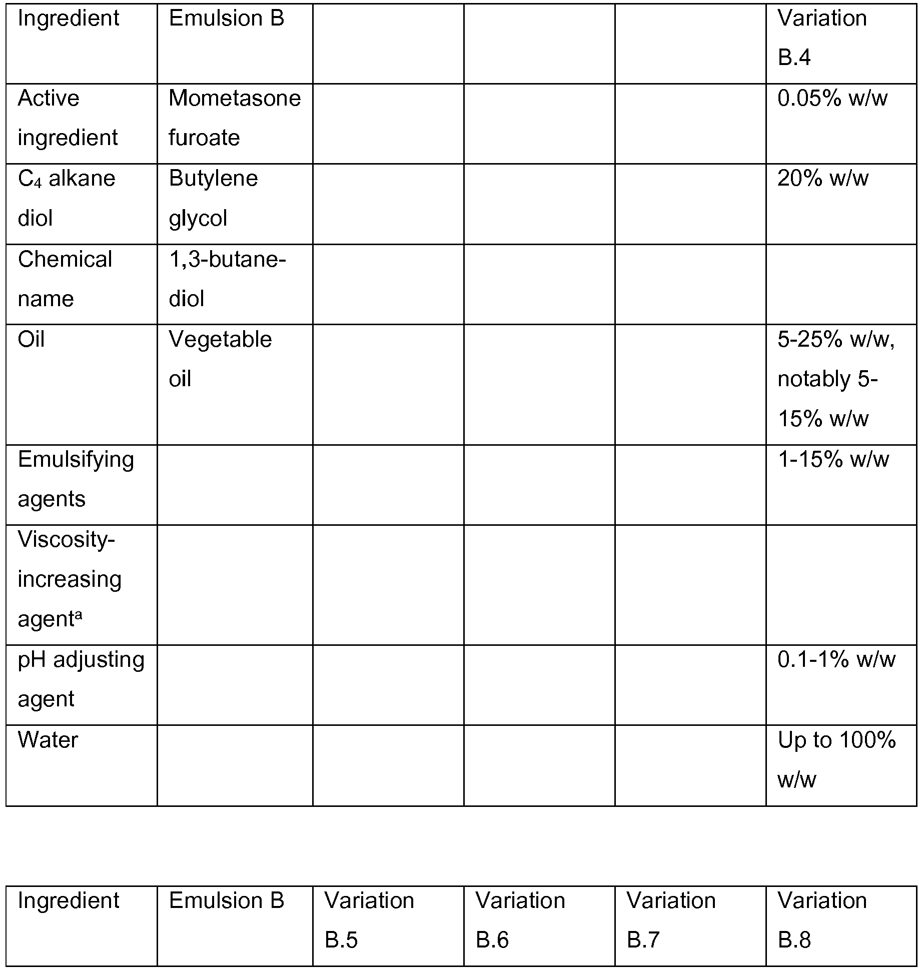

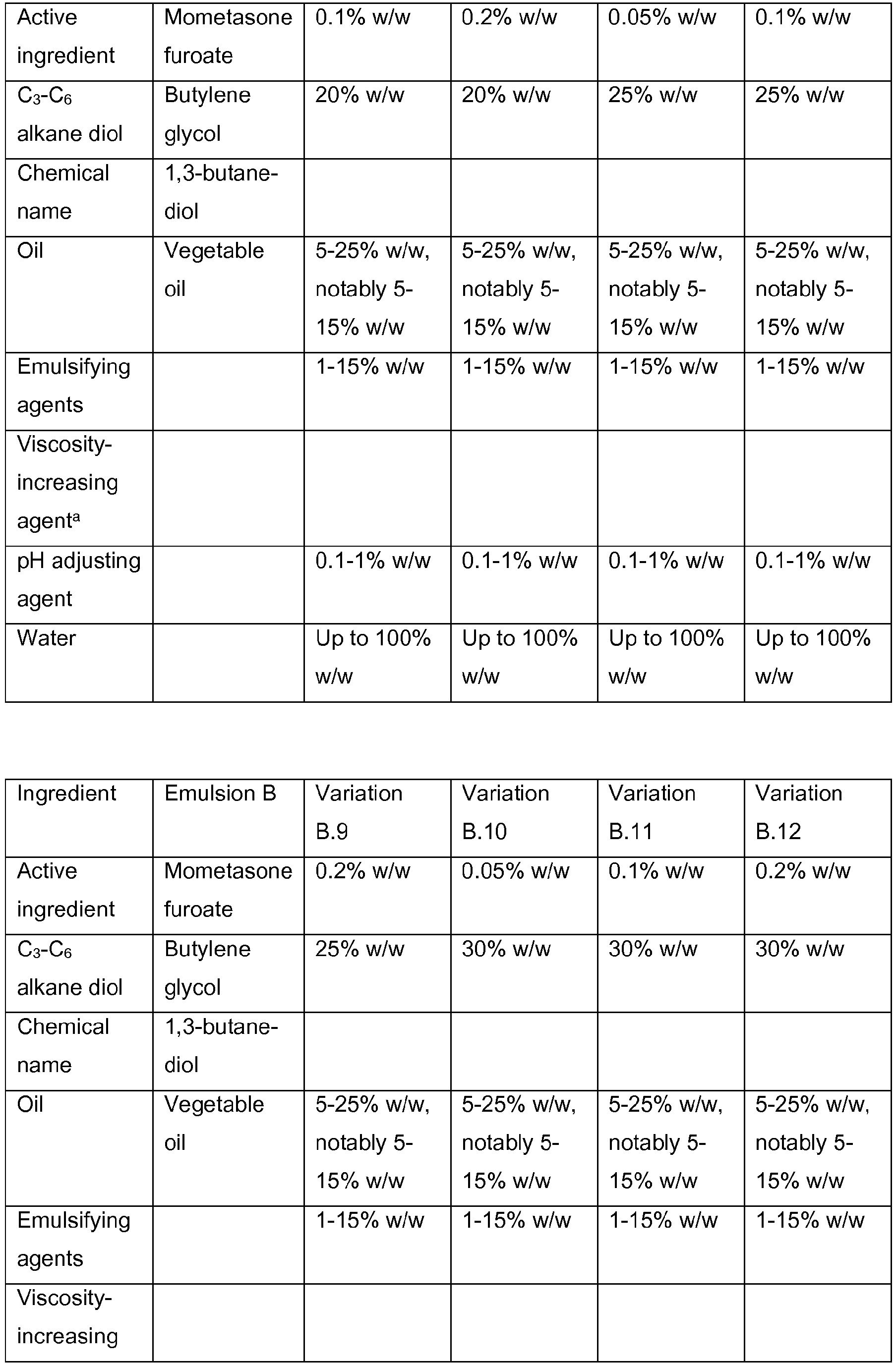

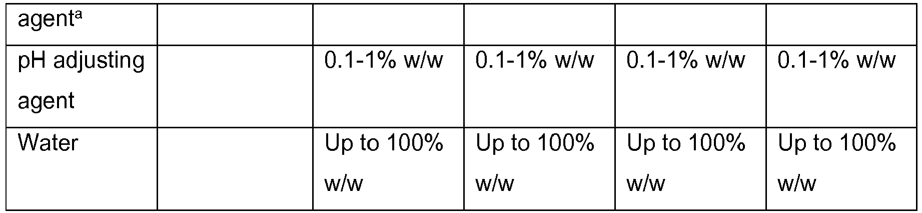

^{a} the concentration of the viscosity-increasing agent, if present, depends on the nature of the agent, cf. the text above

Further examples are:
An oil-in-water emulsion according to the invention containing:
   0.05-0.2 % w/w of mometasone or a pharmaceutically acceptable derivative thereof (calculated as mometasone furoate)
   20-45 % w/w of butylene glycol,
   3-30 % w/w of a vegetable oil/fat,
   1-15 % w/w of one or more emulsifying agents,
   optionally 0.1-1% w/w of a pH adjusting agent,
   optionally 5-15% w/w of a viscosity increasing agent,
   up to 100% w/w of water.

An oil-in-water emulsion according to the invention containing:
0.06-0.15 % w/w of mometasone or a pharmaceutically acceptable derivative thereof (calculated as mometasone furoate)
20-40 % w/w of butylene glycol,
5-15 % w/w of a vegetable oil/fat,
1-10 % w/w of one or more emulsifying agents,
0.1-1 % w/w of a pH adjusting agent to adjust pH of the emulsion to about 4-6, optionally 5-15% w/w of a viscosity increasing agent,
up to 100% w/w of water.

An oil-in-water emulsion according to the invention containing:
0.1 % w/w of mometasone or a pharmaceutically acceptable derivative thereof (calculated as mometasone furoate)
from 20 to 30 % w/w of butylene glycol,
from 5 to 10 % w/w of a vegetable oil/fat,
from 5-10 % w/w of one or more emulsifying agents,
0.1-1 % w/w of a pH adjusting agent to adjust pH of the emulsion to about 4-6,
5-10% w/w of a viscosity increasing agent,
up to 100% w/w of water.

Notably, an oil-in-water emulsion according to the invention does not contain N-methyl-2-pyrrolidone.

The present invention also provides a method for manufacturing of an oil-in-water emulsion of the invention. The procedure is detailed described in Example 1 and a person skilled in the art will understand that the individual ingredients mentioned can be replaced by the ingredients mentioned in Table 1 below having the same functionality and in the concentration ranges mentioned. More specifically, the method comprises
i) preparing the oil phase by mixing the ingredients that make up the oil phase and heating to a temperature of from 60 °C to 80 °C, notably from 65 °C to 75 °C such as about 70 °C,
ii) preparing the aqueous phase by a) preparing a dispersion of mometasone or a pharmaceutically acceptable derivative thereof such as mometasone furoate in part of the aqueous phase, b) preparing the remaining part of the aqueous phase by dissolving the ingredients, optionally by heating to 55 °C to 75 °C, notably from 60 °C to 70 °C such as about 65 °C, and c) addition of the dispersion resulting from a) to the remaining part of the aqueous phase resulting from b) to obtain the aqueous phase,
iii) transferring the oil phase i) to the aqueous phase ii) or optionally ii) to i)
iv) mixing until an emulsion is obtained,
v) optionally, subjecting the thus obtained emulsion to vacuum conditions,
vi) optionally, homogenizing the emulsion optionally under vacuum conditions,
vii) optionally, addition of one or more fragrance agents,
viii) cooling the thus obtained emulsion.

The ingredients, included in the oil phase i) above, are typically an oil as described herein and all the ingredients that are soluble in the oil phase (apart from mometasone). Such ingredients may be one or more emulsifying agents, one or more viscosity-increasing agents, one or more preservatives, if present, optionally one or more fragrance agents or the like. The ingredients included in the aqueous phase is - apart from mometasone - water and ingredients that are soluble in water such as e.g. one or more C₃-C₆ alkane-diols, one or more pH regulating agents, if present, one or more water-soluble viscosity-increasing agents, if present, one or more preservatives, if present, optionally one or more fragrance agents, or the like.

The invention is further illustrated in the following Figures and Examples without limiting the invention thereto.

### Figures

Figure 1 shows skin blanching as a function of time for the oil-in-water cream with a composition given in Example 1, containing 25% propylene glycol, and the commercial water-in-oil cream Elocon® 0.1 % cream. The skin is exposed to the creams 5 hours prior to evaluation (results from Example 2).
Figure 2 shows skin blanching as a function of time for oil-in-water creams, containing 20% and 30% propylene glycol, and the commercial water-in-oil cream Elocon® 0.1 % cream. The skin is exposed to the creams 6 hours prior to evaluation (results from Example 2).
Figure 3 shows skin blanching as a function of time for oil-in-water creams containing 20% oil/fat mainly coconut oil (includes all ingredients with HLB≤11) and 20% propylene glycol (90016-0712-14); 40% oil/fat mainly paraffin oil and white soft paraffin (includes all ingredients with HLB≤11) and no propylene glycol (90016-0711-47). The skin is exposed to the creams 6 hours prior to evaluation (results from Example 2).
Figure 4. Mean AUC of baseline-corrected, untreated control site-corrected values and 95% confidence intervals (Per Protocol Population) - results from Example 2.
Figure 5 shows skin blanching as a function of time for oil-in-water creams containing 25% w/w propylene glycol, butylene glycol, and hexylene glycol, respectively. The compositions are as described in Table 1 using the relevant alkane diol.
Figure 6 shows skin blanching as a function of time for oil-in-water creams containing i) 25% propylene glycol and 20% coconut oil (weight ratio PG:W of 1:1.58) and ii) the composition described in table 1 herein (PG:W of 1:2.06), and the commercial water-in-oil cream Elocon® 0.1 % cream. The skin is exposed to the creams 6 hours prior to evaluation - results from Reference Example 3.
Figure 7 shows skin blanching as a function of time for a cream according to the invention compared with Elocon® 0.1 % cream and creams prepared according to examples 3 and 5, respectively, of WO 2008/126076 (in short: Perrigo creams). PG:W of Perrigo creams is 1:3.6.

### Examples

### Example 1

### Complete composition

The complete composition of the 0.1 % mometasone furoate oil-in-water cream is given in table 1.

**Table 1. Complete composition of 0.1 % mometasone furoate oil-in-water cream.**

| **Name of ingredient** | **Quantity (mg/g)** | **Function** | **Concentration range (mg/g)** | **Replacement** |
|---|---|---|---|---|
| **Active substance** | | | | |
| Mometasone furoate, micronized 100% ≤ 20 µm 99%≤15 µm 80% ≤ 5 µm | 1.0 | Drug substance | 0.10-2.0 | - |

| **Excipients** | | | | |
|---|---|---|---|---|
| Coconut oil | 80 | Emollient | 30 - 300 | Vegetable oil/fat, animal oil/fat, mineral oil, ester oil or wax, silicon oil |
| Stearic acid | 20 | Emulsifying agent | 10-50 | glycerol alkyl esters, macrogol alkyl esters, polyoxyethyleneglycol alkyl esters, fatty acids, polyoxyethylene sorbitan esters, polyoxyethylene alkyl ethers, galactolipids |
| Macrogol Stearate | 30 | Emulsifying agent Solubilizer of drug substance | 10-50 | |
| Glycerol monostearate 40-55 | 30 | Emulsifying agent | 10-50 | |
| Cetostearyl alcohol | 70 | Viscosity-increasing agent | 50-150 | Fatty alcohols |
| Alkane diol, notably Propylene glycol or butylene glycol | 250 | Solvent Solubilizer of drug substance | 150-450 | propylene glycol, butylene glycol, pentylene glycol, hexylene glycol |
| Sodium citrate | 2.7 | pH regulating agent | 1-10 | hydrochloric acid, phosphoric acid, sodium hydroxide, citrate buffer, phosphate buffer, phthalate buffers, acetate buffers, succinate buffers |
| Citric acid, monohydrate | 2.5 | pH regulating agent | | |
| Water, purified | Ad 1g | Solvent | - | - |

### Description of manufacturing process

Mometasone furoate is dispersed in a small portion of an alkane diol notably propylene glycol or butylene glycol and water mixture. The remaining parts of alkane diol notably propylene glycol or butylene glycol and water are mixed and heated to about 65°C together with sodium citrate and citric acid. Thereafter, the dispersion of mometasone furoate is added to the aqueous phase.

The ingredients of the oil phase (coconut oil, stearic acid, cetostearyl alcohol, macrogol stearate) are mixed and heated to about 70°C.

The oil phase is added to the aqueous phase. The emulsion is mixed and homogenized and thereafter the cream is cooled during stirring.

### Detailed description of manufacturing process

1. Mometasone furoate dispersion.
   *Mix a small, suitable amount of 2 parts purified water and 1 part alkane diol notably propylene glycol or butylene glycol in a vessel. Add mometasone furoate to the vessel. Stir the mixture for 5 minutes, until the active substance is dispersed.*
2. Mixing and heating of water phase to clear solution.
   *Add sodium citrate, citric acid monohydrate, alkane diol notably propylene glycol or butylene glycol and purified water to a vessel. Heat to 65°C* ± *5°C during mixing at low speed. Control that a clear solution is obtained.*
3. Addition of the mometasone furoate dispersion to the water phase. *Add the dispersed active substance to the water phase.*
4. Add raw material to oil phase.
   *Add all the raw materials of the oil phase to a vessel.*
5. Melting and heating of oil phase
   *Heat and melt the oil phase to 70°C* ± *5°C.*
6. Mixing to homogeneous solution.
   *Control that the oil phase is a homogeneous solution.*
7. Transfer the oil phase to the water phase.
   *Transfer all the oil phase to the water phase.*
8. Mixing.
   *Mix the two phases until a white emulsion has emerged. Use medium mixing speed.*
9. Add vacuum.
   *Carefully add vacuum.*
10. Homogenisation.
   *Homogenize the emulsion for about 5 - 10 minutes, while stirring.*
11. Cooling.
   *Cool the emulsion while stirring, until the temperature of the emulsion reaches 25°C.*
12. Final mixing.
   *When the cream (emulsion) has reached a temperature of 25°C, mix for additional 15 minutes at low speed.*

### Stability data

After storage at 25°C/60% RH and 40°C/75% RH for up to 9 months, the 0.1 % mometasone furoate oil-in-water cream with 25% propylene glycol is stable both chemically and physically, see table 2 and 3.

Samples with the composition according to table 1 but with 20 % (w/w) propylene glycol have been stored at 25°C/60%RH and 40°C/75%RH. Stability data is available for up to 12 months, see table 4 and 5. Data shows that the composition is stable at both temperatures for the investigated period.

**Table 2**

| Stability data after storage of the composition according to table 1 with propylene glycol, Mometasone furoate 0.1 % cream, batch No. 90016-0806-07, at 25°C/60% RH in aluminum laminated co-extruded PE 100 g tubes. | | | | | | | |
|---|---|---|---|---|---|---|---|
| | | | | **Related substances** | | | |
| **Storage Months** | **Appearance** | **pH** | **Assay, mometasone furoate % (m/m)** | **Unknown RRT=0.77 area-%** | **Unknown RRT=1.03 area-%** | **Unknown RRT=1.07 area-%** | **Sum of related substances area-%** |
| 0 | Complies | 4.7 | 0.098 | <0.10 | <0.10 | 0.10 | 0.10 |
| 3 | Complies | 4.5 | 0.098 | <0.10 | <0.10 | 0.11 | 0.11 |
| 6 | Complies | 4.6 | 0.099 | <0.10 | <0.10 | 0.11 | 0.11 |
| 9 | Complies | 4.6 | 0.099 | <0.10 | <0.10 | 0.11 | 0.11 |

**Table 3**

| Stability data after storage of the composition according to table 1 with propylene glycol, Mometasone furoate 0.1 % cream, batch No. 90016-0806-07, at 40°C/75% RH in aluminum laminated co-extruded PE 100 g tubes. | | | | | | | |
|---|---|---|---|---|---|---|---|
| | | | | **Related substances** | | | |
| **Storage Months** | **Appearance** | **pH** | **Assay, mometasone furoate % (m/m)** | **Unknown RRT=0.77 area-%** | **Unknown RRT=1.03 area-%** | **Unknown RRT=1.07 area-%** | **Sum of related substances area-%** |
| 0 | Complies | 4.7 | 0.098 | <0.10 | <0.10 | 0.10 | 0.10 |
| 3 | Complies | 4.5 | 0.097 | <0.10 | 0.12 | 0.10 | 0.22 |
| 6 | Complies | 4.5 | 0.098 | <0.10 | 0.18 | 0.10 | 0.28 |

**Table 4**

| Stability data after storage of the composition according to table 1 but with 20 % (w/w) propylene glycol, Mometasone furoate 0.1% cream, batch No. 90016-0712-14, at 25°C/60% RH in 30 g aluminum tubes. | | | | | | | |
|---|---|---|---|---|---|---|---|
| | | | | **Related substances** | | | |
| **Storage Months** | **Appearance** | **pH** | **Assay, mometasone furoate % (m/m)** | **Unknown RRT=0.77 area-%** | **Unknown RRT=1.03 area-%** | **Unknown RRT=1.07 area-%** | **Sum of related substances area-%** |
| 0 | Complies | 4.6 | 0.100 | <0.10 | <0.10 | <0.10 | <0.10 |
| 1 | n.a. | n.a. | n.a. | n.a. | n.a. | n.a. | n.a. |
| 4 | Complies | 4.6 | 0.103 | <0.10 | <0.10 | <0.10 | <0.10 |
| 6 | Complies | 4.8 | 0.100 | <0.10 | <0.10 | 0.10 | 0.10 |
| 12 | Complies | 4.6 | 0.102 | <0.10 | <0.10 | 0.11 | 0.11 |

| | | | | | | | |
|---|---|---|---|---|---|---|---|
| n.a.= not analysed | | | | | | | |

**Table 5**

| Stability data of the composition according to table 1 but with 20 % (w/w) propylene glycol, Mometasone furoate 0.1% cream, batch No. 90016-0712-14, after storage at 40°C/75% RH in 30 g aluminum tubes. | | | | | | | |
|---|---|---|---|---|---|---|---|
| | | | | **Related substances** | | | |
| **Storage Months** | **Appearance** | **pH** | **Assay, mometasone furoate % (m/m)** | **Unknown RRT=0.77 area-%** | **Unknown RRT=1.03 area-%** | **Unknown RRT=1.07 area-%** | **Sum of related substances area-%** |
| 0 | Complies | 4.6 | 0.100 | <0.10 | <0.10 | <0.10 | <0.10 |
| 1 | Complies | 4.7 | 0.101 | <0.10 | <0.10 | <0.10 | <0.10 |
| 3 | Complies | 4.7 | 0.105 | <0.10 | <0.10 | <0.10 | <0.10 |
| 6 | Complies | 4.8 | 0.100 | <0.10 | 0.16 | 0.10 | 0.26 |

### Example 2

### In vivo behavior of oil-in-water emulsions

### VCA Screening Study

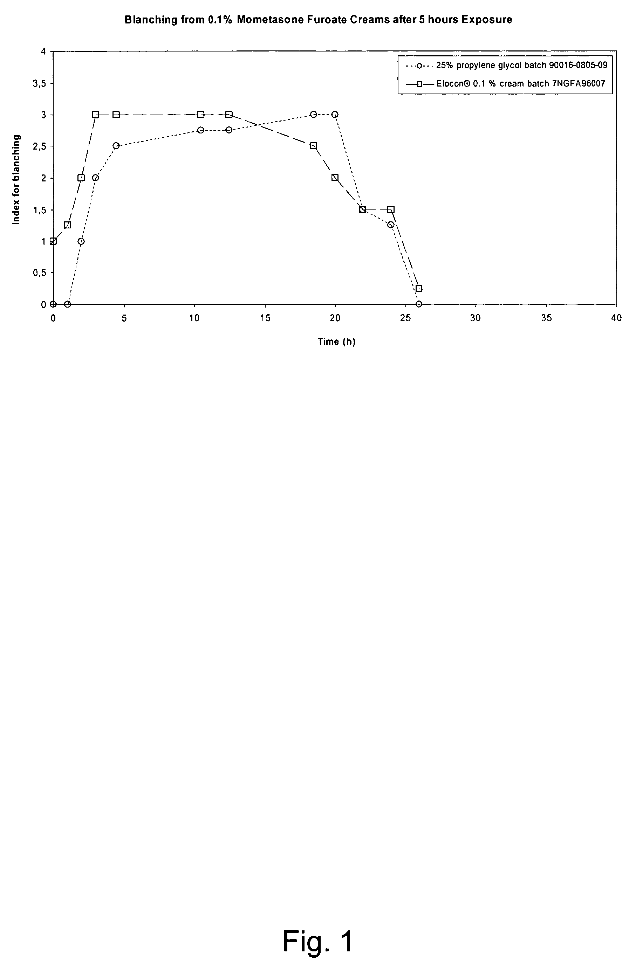

Skin blanching has been evaluated to assess the topical bioavailability of the 0.1 % mometasone furoate oil-in-water cream given in Example 1 with propylene glycol, see Figure 1. The blanching effect has been compared to Elocon® 0.1 % cream. Elocon® 0.1 % cream is a water-in-oil emulsion, containing 0.1 % mometasone furoate. In figure 1, it can be seen that the skin blanching and thereby the bioavailability of the 0.1 % mometasone furoate oil-in-water cream is comparable to that of Elocon® 0.1 % cream.

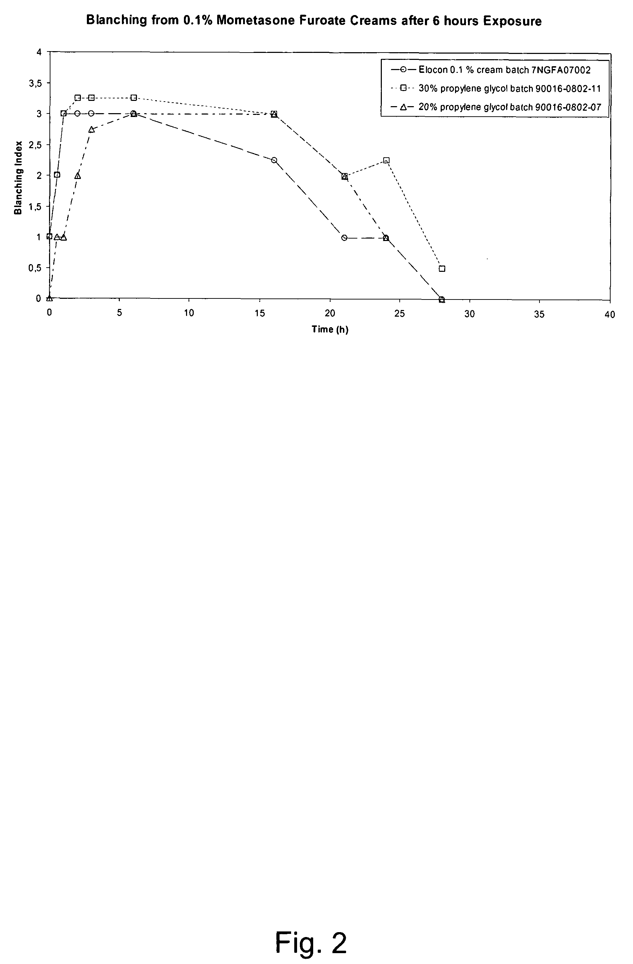

In figure 2, skin blanching after applying 0.1 % mometasone furoate oil-in-water creams containing 20 % and 30 % propylene glycol was compared with Elocon® 0.1 % cream. Also for these creams the skin blanching was comparable to that of Elocon® 0.1 % cream. The creams employed has an alkane diol and water ratio of from 1:1.5 to 1:2.8.

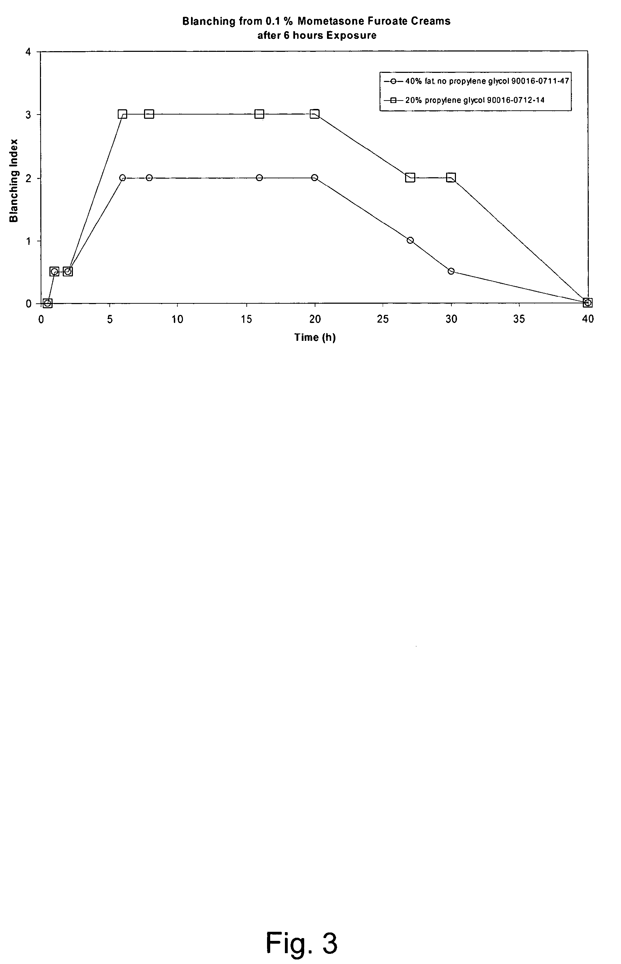

The effect on skin blanching after applying an oil-in-water cream with a high fat content (40 %) and no propylene glycol was compared with the effect of a cream with 20 % propylene glycol and 20 % fat, see figure 3. It can be seen that the presence of propylene glycol in the formulations is more important than a high fat content.

In the in-vivo screening studies, approximately 40 mg of each cream was applied to a 2.25 cm² test field located on the volar part of the forearm. The non-occlusive application was removed after 5 hours (figure 1) or 6 hours (figure 2 and 3) exposure of the creams. Prior to the creams were applied, the skin color was determined by comparing the color of the test field with shades in a color atlas (Natural Color System SS019102, 2nd ed.). White is given an index of 4 (color 0502-Y) and untreated skin color on this object gave an index of 0 (color S10-C20-Y50R).

### VCA Study

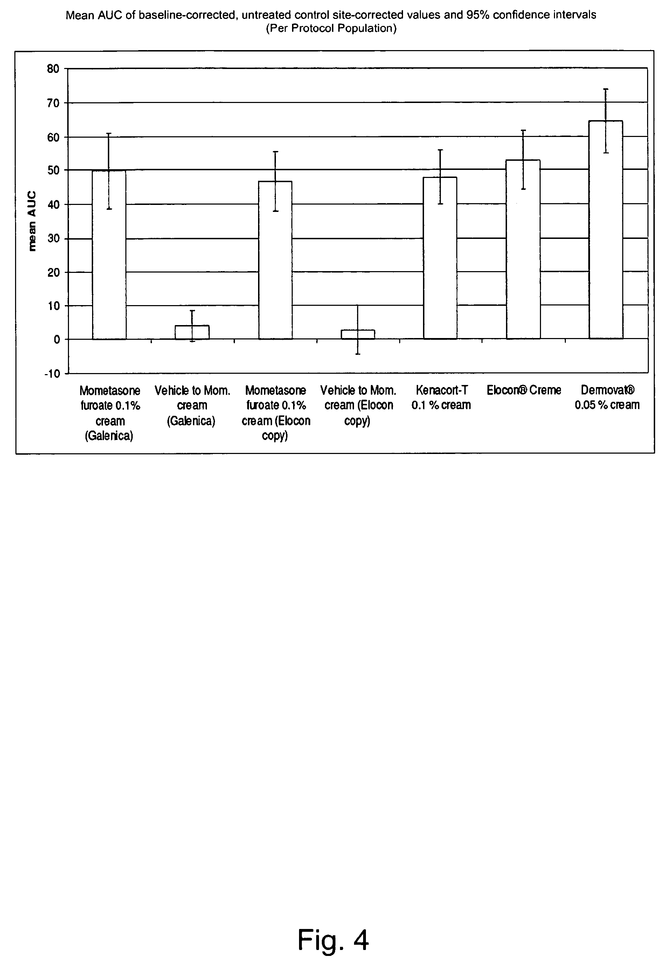

A vasoconstrictor assay (VCA) study on 30 healthy subjects, vehicle controlled single-center double blind study for the study preparations (Mometasone furorate 0.1 % cream, Galenica (the invention) and Elocon generic copy, class III) observer-blind for the comparators (Kenacort-T 0.1 % cream, Elocon^{®} 0.1% cream and Dermovat 0.05% cream, class II, III and IV respectively) was performed. Approximately 50 µl cream was applied to a total of nine test fields of 2 cm² each, located on the volar surface of the forearms, non-occlusive for 6 hours. The skin color was measured prior to treatment (baseline) and after 1, 2, 4, 6, 18 and 24 hours after the end of the treatment period with a Minolta Chroma-Meter CR-300. The total mean skin blanching was assessed as baseline corrected AUC for the tested creams according to figure 4.

The result shows that topical bioequivalence is possible to obtain with the described o/w cream when compared to the Elocon^{®} w/o cream.

### Antimicrobial Properties

The 0.1 % mometasone furoate oil-in-water cream has been challenge tested according to Ph. Eur. "Efficacy of Antimicrobial Preservation". The o/w cream showed that the formulation satisfies criteria in Ph. Eur. 5.1.3. Hence, the product is self preserved and has antimicrobial properties.

### Oil-in-water emulsions with content of different alkane-diols

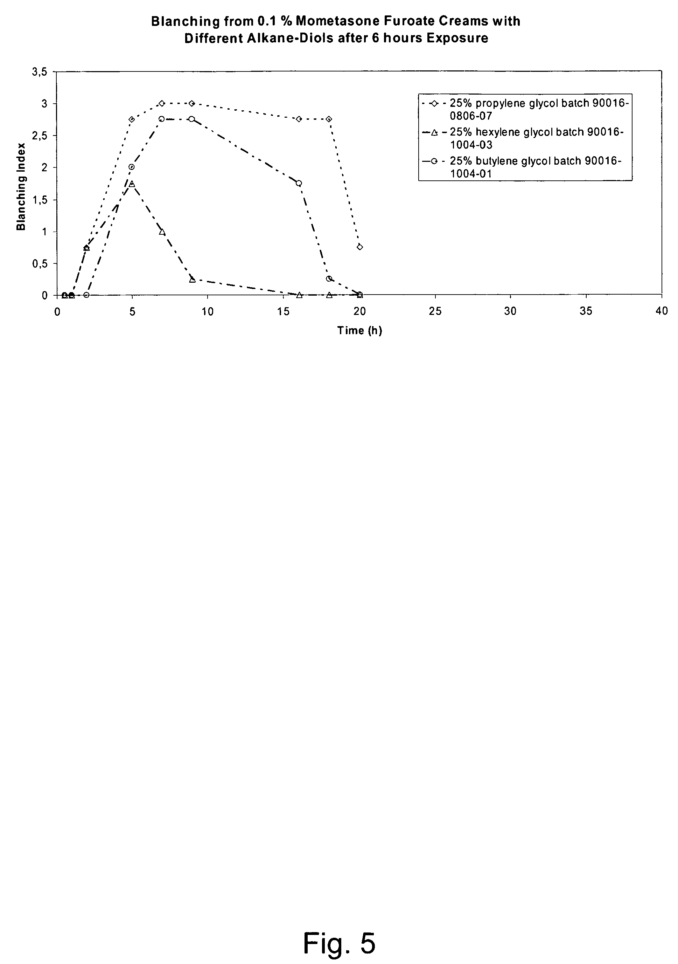

Three oil-in-water emulsions having a content of propylene glycol, butylene glycol, and hexylene glycol were tested as described above. The results at shown in Figure 5. From this figure it is seen that only propylene glycol and butylene glycol leads to a therapeutic effect that is similar or very close to that of Elocon® cream (see also figure 2 for Elocon® data).

### Reference Example 3

### In vivo behavior of the oil-in-water emulsions

Two compositions containing propylene glycol as alkane diol and Elocon® 0.1% were tested as described in Example 2 above. The compositions of the invention had different weight ratios, alkane-diol: water, PG:W, namely from 1:1.6 to 1:2.1. The composition with the weight ratio 1:1.6 was in accordance with the composition described in Table 1 herein, but contained 20% w/w coconut; the content of water was reduced accordingly. The composition with the weight ratio 1:2.1 contained 25% propylene glycol and had a composition as described in Table 1 herein.

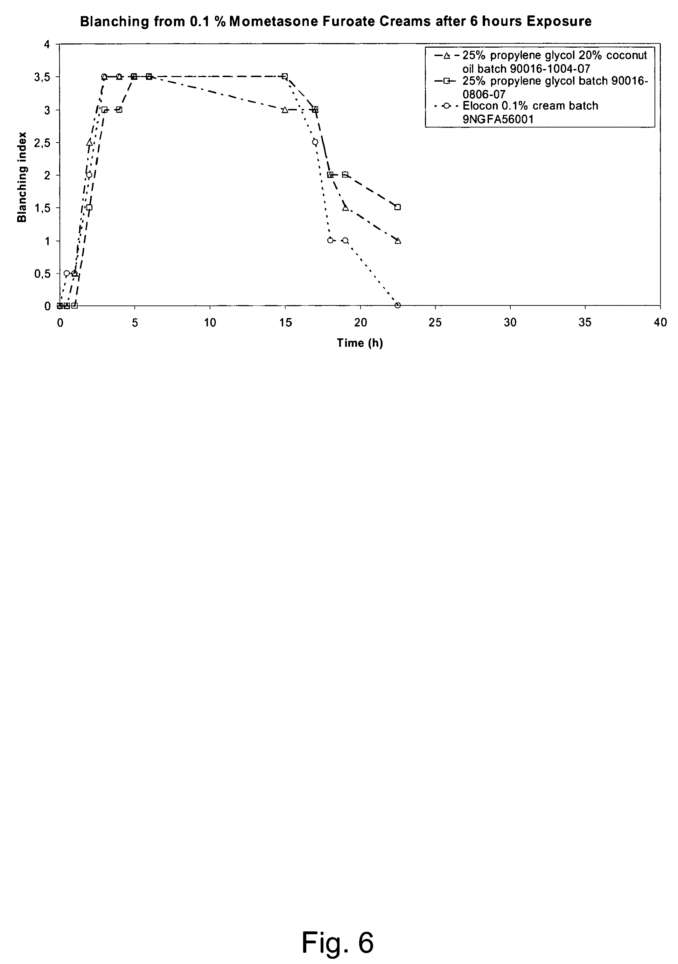

The results show that both compositions have therapeutic effects similar to that of Elocon® cream (see figure 6).

### Reference Example 4

### Comparison of in vivo behaviour of an oil-in-water emulsion with formulations according to WO 2008/126076 (Perrigo Israel Pharmaceuticals Ltd)

As mentioned in the introduction herein, an oil-in-water composition of mometasone has been described in WO 2008/126076. However, no *in vivo* studies have been reported. In order to compare the *in vivo* behaviour of compositions according to the present invention with those of WO 2008/126076 (denoted Perrigo creams), a comparison study was made.

The compositions described in Example 3 (Formula B) and Example 5 (Formula C) were prepared as follows:
Composition Perrigo Mometasone furoate 0.1 % cream (comparative cream according to WO 2008/126076)

| Formula B (Comparative) in patent WO2008/126076 | |
|---|---|
| **Ingredients** | **Concentration (wt%)** |
| Mometasone furoate | 0.1 |
| Phosphoric acid | 0.525 |
| Purified water | 53.7 |
| Xanthan gum | 0.2 |
| Carbomer 940 | 0.3 |
| Dibasic sodium phosphate anhydrous (corresponding to 1.0 wt % heptahydrate) | 0.53 |
| Emulsifying wax (Polawax) | 8.0 |
| Benzyl alcohol | 1.0 |
| Propylene glycol | 15.0 |
| Cetostearyl alcohol | 7.0 |
| Oleic acid | 1.2 |
| Caprylic capric triglyceride | 12.0 |

| Formula C (Invention) in patent WO2008/126076 | |
|---|---|
| **Ingredients** | **Concentration (wt%)** |
| Mometasone furoate | 0.075 |
| Xanthan gum | 0.2 |
| Carbomer 940 | 0.3 |
| Dibasic sodium phosphate anhydrous (corresponding to 1.0 wt % heptahydrate) | 0.53 |
| Emulsifying wax (Polawax) | 8.0 |
| Benzyl alcohol | 1.0 |
| Propylene glycol | 15.0 |
| Cetostearyl alcohol | 7.0 |
| Oleic acid | 1.2 |
| Caprylic capric triglyceride | 12.0 |
| Phosphoric acid | 0.525 |
| Purified water | 53.7 |

### Preparation of creams:

The water phase is prepared first: Xanthan gum and carbomer 940 are dispersed in purified water. Next dibasic sodium phosphate is mixed into the dispersion. Emulsifying wax and benzyl alcohol are added to the dispersion and heated.

To prepare the active solution, mometasone furoate is dissolved in heated propylene glycol.

Next the oily phase is prepared: Oleic acid, cetostearyl alcohol, and caprylic capric triglyceride are combined and mixed.

The active solution and the oily phase are added to the water phase.

The resulting emulsion is cooled. pH is adjusted with phosphoric acid.

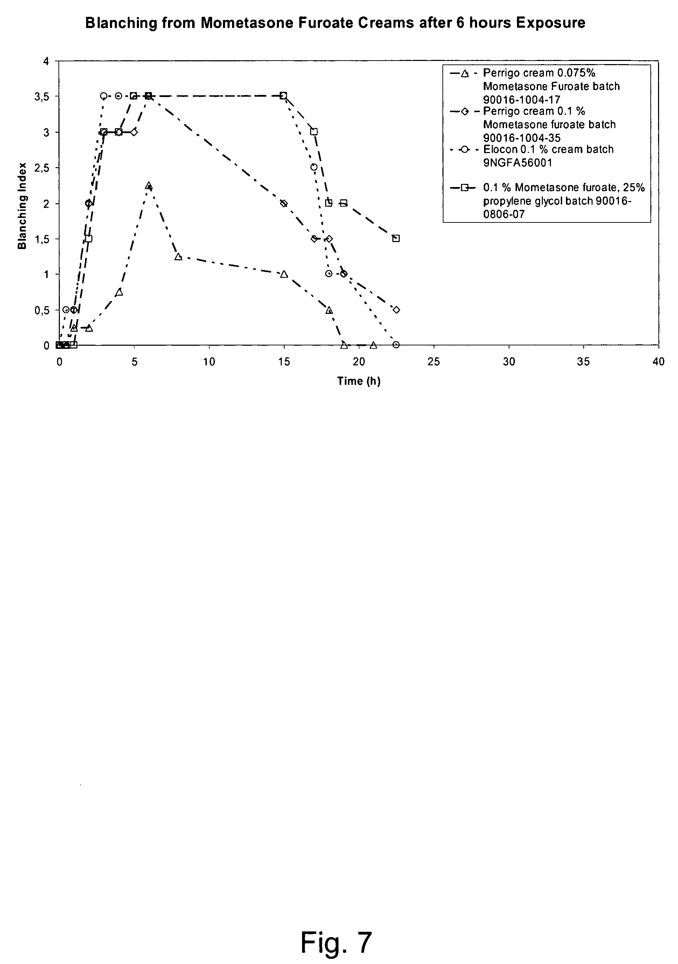

The Perrigo compositions and an oil-in-water composition in accordance with the present invention, but containing propylene glycol, and Elocon® 0.1 % cream were subjected to the skin blanching study described in Example 2 above and the results are reported in Figure 7. The results clearly demonstrate that the Perrigo creams do not lead to similar therapeutic effect as the Elocon® cream, whereas a composition of the invention has similar therapeutic effect as Elocon® cream.

## Claims

1. An oil-in-water emulsion comprising mometasone or a pharmaceutically acceptable derivative thereof and a C₄ alkane-diol, which may be linear or branched, and the concentration of the C₄ alkane-diol is from 20 to 45% w/w.

2. An oil-in-water emulsion according to any of the preceding claims, wherein the C₄ alkane-diol is butylene glycol, such as 1,3-butane-diol.

3. An oil-in-water emulsion according to claim 1, wherein the concentration of butylene glycol is from 20 to 40% w/w such as from 20 to 30% w/w.

4. An oil-in-water emulsion according to claim 1 or 2, wherein the weight ratio between the butylene glycol and water contained in the oil-in-water emulsion is from 1:1 to 1:3.

5. An oil-in-water emulsion according to any of the preceding claims, wherein mometasone or a pharmaceutically acceptable derivate thereof is the sole therapeutically active ingredient.

6. An oil-in-water emulsion according to any of the preceding claims, wherein a part of mometasone or a pharmaceutically acceptable derivative thereof is present in undissolved form.

7. An oil-in-water emulsion according to any of the preceding claims, wherein mometasone is present in the emulsion in micronised form.

8. An oil-in-water emulsion according to any of the preceding claims, wherein the oil phase comprises an oil selected from a group consisting of vegetable oils, animal oils, mineral oils, ester oils, and silicon oils.

9. An oil-in-water emulsion according to any of the preceding claims further comprising one or more emulsifying agents.

10. An oil-in-water emulsion according to claim 9, wherein the one or more emulsifying agents are selected from the group consisting of glycerol alkyl esters, macrogol alkyl esters, polyoxyethyleneglycol alkyl esters, fatty acids, polyoxyethylene sorbitan esters, polyoxyethylene alkyl ethers, and galactolipids.

11. An oil-in-water emulsion according to the preceding claims further comprising a viscosity-increasing agent.

12. An oil-in-water emulsion according to claim 9 or 10, wherein the one or more viscosity-increasing agent is cetostearyl alcohol.

13. An oil-in-water emulsion according to any of the preceding claims containing:
0.05-0.2 % w/w of mometasone or a pharmaceutically acceptable derivative thereof (calculated as mometasone furoate) 20-45 % w/w of butylene glycol,
3-30 % w/w of a vegetable oil,
1-15 % w/w of one or more emulsifying agents,
optionally 0.1-1% w/w of a pH adjusting agent,
optionally 5-15% w/w viscosity increasing agent,
up to 100% w/w of water.

14. An oil-in-water emulsion according to any of the preceding claims with the proviso that the emulsion does not contain N-methyl-2-pyrrolidone.

15. An oil-in-water emulsion according to any of the preceding claims with the proviso that the emulsion does not contain chlorocresol.

## Patentansprüche

1. Eine Öl-in-Wasser-Emulsion, umfassend Mometason oder ein pharmazeutisch annehmbares Derivat davon und ein C4-Alkan-Diol, das linear oder verzweigt sein kann, und die Konzentration des C4-Alkan-Diols ist von 20 bis 45 Gewichtsprozent.

2. Eine Öl-in-Wasser-Emulsion gemäß einem der vorhergehenden Ansprüche, wobei das C4-Alkan-Diol Butylenglycol ist, wie etwa 1,3-Butandiol.

3. Eine Öl-in-Wasser-Emulsion gemäß Anspruch 1, wobei die Konzentration von Butylenglycol von 20 bis 40 Gewichtsprozent, wie etwa von 20 bis 30, Gewichtsprozent beträgt.

4. Eine Öl-in-Wasser-Emulsion gemäß Anspruch 1 oder 2, wobei das Gewichtsverhältnis zwischen dem Butylenglycol und dem in der Öl-in-Wasser-Emulsion enthaltenen Wasser von 1:1 bis 1:3 beträgt.

5. Eine Öl-in-Wasser-Emulsion gemäß einem der vorhergehenden Ansprüche, wobei Mometason oder ein pharmazeutisch annehmbares Derivat davon der einzige therapeutisch aktive Bestandteil ist.

6. Eine Öl-in-Wasser-Emulsion gemäß einem der vorhergehenden Ansprüche, wobei ein Teil des Mometasons oder pharmazeutisch annehmbaren Derivats davon in ungelöster Form vorliegt.

7. Eine Öl-in-Wasser-Emulsion gemäß einem der vorhergehenden Ansprüche, wobei Mometason in der Emulsion in mikronisierter Form vorliegt.

8. Eine Öl-in-Wasser-Emulsion gemäß einem der vorhergehenden Ansprüche, wobei die Öl-Phase ein Öl umfasst, das ausgewählt ist aus der Gruppe bestehend aus pflanzlichen Ölen, tierischen Ölen, Mineralölen, Esterölen und Silikonölen.

9. Eine Öl-in Wasser-Emulsion gemäß einem der vorhergehenden Ansprüche, die außerdem ein oder mehrere emulgierende Agentien umfasst.

10. Eine Öl-in Wasser-Emulsion gemäß Anspruch 9, wobei das eine oder die mehreren emulgierenden Agentien ausgewählt sind aus der Gruppe bestehend aus Glycerin-Alkylestern, Macrogol-Alkylestern, Polyozyethylenglycol-Alkylestern, Fettsäuren, Polyozyehtylen-Sorbitanestern, Polyoxyethylen - Alkylethern und Galactolipiden.

11. Eine Öl-in-Wasser-Emulsion gemäß einem der vorhergehenden Ansprüche, die außerdem ein viskositätssteigerndes Agens umfasst.

12. Eine Öl-in-Wasser-Emulsion gemäß Anspruch 9 oder 10, wobei das eine oder mehrere viskositätssteigernde Agens Cetylstearylalkohol ist.

13. Eine Öl-in-Wasser-Emulsion gemäß einem der vorhergehenden Ansprüche, enthaltend:
0,05-0,2 Gewichtsprozent Mometason oder eines pharmazeutisch annehmbaren Derivats davon (berechnet als Mometasonfuroat)
20-45 Gewichtsprozent Butylenglycol,
3-30 Gewichtsprozent eines pflanzlichen Öls,
1-15 Gewichtsprozent eines oder mehrerer emulgierender Agentien,
optional 0,1-1 Gewichtsprozent eines pH-einstellenden Agens,
optional 5-15 Gewichtsprozent eines viskositätssteigernden Agens,
bis zu 100 Gewichtsprozent Wasser

14. Eine Öl-in-Wasser-Emulsion gemäß einem der vorhergehenden Ansprüche unter der Bedingung, dass die Emulsion kein N-Methyl-2-pyrrolidon enthält.

15. Eine Öl-in-Wasser-Emulsion gemäß einem der vorhergehenden Ansprüche, unter der Bedingung, dass die Emulsion kein Chlorkresol enthält.

## Revendications

1. Émulsion huile-dans-eau comprenant de la mométasone ou un dérivé pharmaceutiquement acceptable de celle-ci et un alcane-diol en C₄, qui peut être linéaire ou ramifié, et la concentration de l'alcane-diol en C₄ est de 20 à 45 % en p/p.

2. Émulsion huile-dans-eau selon l'une quelconque des revendications précédentes, dans laquelle l'alcane-diol en C₄ est un butylène glycol, tel que le 1,3-butane-diol.

3. Émulsion huile-dans-eau selon la revendication 1, dans laquelle la concentration de butylène glycol est de 20 à 40 % en p/p, telle que de 20 à 30 % en p/p.

4. Émulsion huile-dans-eau selon la revendication 1 ou 2, dans laquelle le rapport pondéral entre le butylène glycol et l'eau contenus dans l'émulsion huile-dans-eau est de 1/1 à 1/3.

5. Émulsion huile-dans-eau selon l'une quelconque des revendications précédentes, dans laquelle la mométasone ou un dérivé pharmaceutiquement acceptable de celle-ci est le seul principe thérapeutiquement actif.

6. Émulsion huile-dans-eau selon l'une quelconque des revendications précédentes, dans laquelle une partie de la mométasone ou d'un dérivé pharmaceutiquement acceptable de celle-ci est présente sous forme non dissoute.

7. Émulsion huile-dans-eau selon l'une quelconque des revendications précédentes, dans laquelle la mométasone est présente dans l'émulsion sous forme micronisée.

8. Émulsion huile-dans-eau selon l'une quelconque des revendications précédentes, dans laquelle la phase huileuse comprend une huile choisie dans un groupe constitué des huiles végétales, des huiles animales, des huiles minérales, des huiles d'ester et des huiles de silicium.

9. Émulsion huile-dans-eau selon l'une quelconque des revendications précédentes comprenant en outre un ou plusieurs agents émulsifiants.

10. Émulsion huile-dans-eau selon la revendication 9, dans laquelle le ou les agents émulsifiants sont choisis dans le groupe constitué des esters alkyliques de glycérol, des esters alkyliques de macrogol, des esters alkyliques de polyoxyéthylèneglycol, des acides gras, des esters de polyoxyéthylène sorbitane, des éthers alkyliques de polyoxyéthylène et des galactolipides.

11. Émulsion huile-dans-eau selon les revendications précédentes comprenant en outre un agent augmentant la viscosité.

12. Émulsion huile-dans-eau selon la revendication 9 ou 10, dans laquelle le ou les agents augmentant la viscosité est/sont l'alcool cétostéarylique.

13. Émulsion huile-dans-eau selon l'une quelconque des revendications précédentes contenant :
de 0,05 à 0,2 % en p/p de mométasone ou d'un dérivé pharmaceutiquement acceptable de celle-ci (calculé sous la forme de furoate de mométasone) de 20 à 45 % en p/p de butylène glycol,
de 3 à 30 % en p/p d'une huile végétale,
de 1 à 15 % en p/p d'un ou plusieurs agents émulsifiants,
facultativement de 0,1 à 1 % en p/p d'un agent d'ajustement du pH,
facultativement de 5 à 15 % en p/p d'un agent augmentant la viscosité,
jusqu'à 100 % en p/p d'eau.

14. Émulsion huile-dans-eau selon l'une quelconque des revendications précédentes à condition que l'émulsion ne contienne pas de N-méthyl-2-pyrrolidone.

15. Émulsion huile-dans-eau selon l'une quelconque des revendications précédentes à condition que l'émulsion ne contienne pas de chlorocrésol.
